(19) Europäisches Patentamt
European Patent Office
Office européen des brevets

(11) Veröffentlichungsnummer : **0 093 948**
**B1**

(12) # EUROPÄISCHE PATENTSCHRIFT

(45) Veröffentlichungstag der Patentschrift : **03.12.86**

(51) Int. Cl.$^4$ : **C 12 N 15/00**, C 12 P 19/34

(21) Anmeldenummer : **83104087.8**

(22) Anmeldetag : **26.04.83**

(54) **Doppelstrangiger Vektor und ihn verwendendes Verfahren.**

(30) Priorität : **07.05.82 DE 3217239**
**10.09.82 DE 3233689**

(43) Veröffentlichungstag der Anmeldung :
**16.11.83 Patentblatt 83/46**

(45) Bekanntmachung des Hinweises auf die Patenterteilung : **03.12.86 Patentblatt 86/49**

(84) Benannte Vertragsstaaten :
**AT BE CH DE FR GB IT LI NL SE**

(56) Entgegenhaltungen :
**US-A- 4 371 625**
**Methods in Enzymology Bd 68 Recombinant DNA (R.Wu ed) 1979 Academic Press New York, USA pp. 250-51**
**Gene Bd. 17 (02.03.1982) S. 79-89**
**Molecular Cloning (T.Mamiatis et al) 1982 Cold Spring Harbor Laboratory, p. 5**

(73) Patentinhaber : **Gesellschaft für Biotechnologische Forschung mbH (GBF)**
**Mascheroder Weg 1**
**D-3300 Braunschweig-Stöckheim (DE)**

(72) Erfinder : **Blöcker, Helmut, Dr.**
**Schinkelstrasse 6**
**D-2000 Hamburg (DE)**
Erfinder : **Frank, Ronald, Dr.**
**Stobenstrasse 2**
**D-3340 Wolfenbüttel (DE)**
Erfinder : **Volckaert, Guido, Dr.**
**Groenstraat 18**
**B-3130 Aarschot (BE)**

(74) Vertreter : **Boeters, Hans Dietrich, Dr. et al**
**Boeters, Bauer & Partner Thomas-Wimmer-Ring 14**
**D-8000 München 22 (DE)**

**EP 0 093 948 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Sequenzanalyse von recht großen DNS-Molekülen ist seit der Entwicklung der Maxam-Gilbert- und Sanger-Coulson-Methoden möglich geworden. Diese Methoden beruhen auf drei Hauptprinzipien :

(1) Die Verwendung eines markierten Nucleosidtriphosphats *in vitro* zur radioaktiven Markierung des Polynucleotids an seinen 5'- oder 3'-Enden oder dazwischen ;

(2) Die Anwendung einer synthetisierenden oder abbauenden Technik auf das zu sequenzierende Polynucleotid zur Bildung von vier oder mehr Gruppen von Fragmenten mit einem fixierten gemeinsamen Ende ;

(3) Die Anwendung einer denaturierenden Polyacrylamidgelelektrophorese, um innerhalb der Gruppen Moleküle entsprechend ihrer Länge zu sortieren und abzubilden.

Die Nucleotidsequenz kann danach durch gegenseitigen Vergleich der verschiedenen Fraktionierungsmuster der vier Gruppen als Ergebnis einer Autoradiographie des Polyacrylamidgels gelesen werden. Jede individuelle Analyse kann die Sequenz eines DNS-Segments von 200 bis 500 Nucleotiden liefern. Diese individuellen Segmente werden danach durch Untersuchung überlappender Strecken bzw. Bereiche klassiert.

Eine Sequenzanalyse in großem Maßstab hängt in erster Linie von der Leichtigkeit der Herstellung der Fragmente ab, die in geeigneter Form von den basenspezifischen Reaktionen analysiert werden. Bei der Sanger-Coulson-Methode ist dieses Problem in glänzender Weise dadurch gelöst worden, indem man statistische DNS-Fragmente in sich von Bakteriophagen M13 ableitenden Vektoren an einer Insertionsstelle in Nachbarschaft zu einer für den Vektor spezifischen Sequenz subkloniert, von der Sequenzierungsreaktionen ausgehen (Primerinitiierte Reparatursynthesen in Gegenwart von Dideoxytriphosphaten). Die einstrangige DNS-Matritze kann durch einfache Manipulationen isoliert werden.

Demgegenüber erfordert die Maxam-Gilbert-Methode ein oder mehrere Gelabtrennungsstufen und fragmentspezifische enzymatische Reaktionen zur Einführung und Abtrennung der radioaktiven Markierung. Daher hängt die Strategie oft von einer bereits vorliegenden Restriktionsspaltstellenübersicht ab, um die Anzahl der einzelnen Analysen zu reduzieren.

Der gegenwärtige Stand der Technik bietet für die Maxam-Gilbert-Methode verschiedene Subklonierungsstrategien ähnlich dem M13-System. Dabei kommen doppelstrangige Vektoren zur Anwendung, die folgendes Fremdmerkmal gemeinsam haben : In Nachbarschaft zu einer Subklonierungsstelle (Insertionsstelle für Fremd-DNS) liegen zwei Restriktionsspaltstellen. Fragmente der zu sequenzierenden Fremd-DNS werden in die Insertionsstelle subkloniert. Die Restriktionsspaltstelle in größerer Nachbarschaft zum subklonierten Fragment dient für die Markierungsreaktion. Dabei werden beide Stränge der DNS markiert. Die andere Restriktionsspaltstelle dient zur Trennung der Markierung. Dadurch entstehen zwei markierte Fragmente. Wird das kleinere Fragment nicht entfernt (z. B. durch Gelelektrophorese), verursacht es eine nicht lesbare Sequenz im unteren Bereich des Sequenzierungsgels. Der Verlust an Sequenzinformation beträgt bei den bisher bekannten Verfahren bis zu 30 Nucleotide.

Alle dem gegenwärtigen Stand der Technik entsprechenden Verfahren erfordern mindestens zwei Restriktionen und eine Markierungsreaktion.

Aufgabe der Erfindung ist es, zu sequenzierende Fremd-DNS oder deren Fragmente in einen doppelstrangigen Vektor einzubauen, der auf einfache Weise einzelmarkiert werden kann, d. h. es soll nur ein Strang der DNS markiert werden. Dadurch sollen sowohl die zweite Restriktion als auch eine Gelabtrennung oder ein Verlust an Sequenzinformation umgangen werden.

Erfindungsgemäß wird dazu ein doppelstrangiger Vektor mit

— einer im Vektor nur einmal vorkommenden Insertionsstelle (a) für Fremd-DNS,

— einer im Vektor nur einmal vorkommenden Markierungsstelle (b) des folgenden Typs vorgesehen, die einzelmarkiert werden kann :

(i) $- X^{3'} \qquad YX^{+} -$

$- X^{+} \; Y^{+}{}_{5'} \qquad X -$ oder

$- X^{3'} \qquad YZ -$

$- X^{+} \; Y^{+}{}_{5'} \qquad Z^{+} -$ oder

(ii) $- X \; M \quad Z^{3'} \qquad N^{+}X^{+} -$

$- X^{+}M^{+}{}_{5'} \qquad Z^{+}N \; X -$ oder

$- X\ M\ \ Z^{3'} \qquad N^{+}Y\ -$

$- X^{+}M^{+}{}_{5'} \qquad Z^{+}N\ Y^{+}\ -$ oder

(iii) $- X\ M\ ^{3'} \qquad N\ X^{+}\ -$

$- X^{+}M^{+}{}_{5'} \qquad N^{+}X\ -$ oder

$- M\ X\ ^{3'} \qquad X^{+}N\ -$

$- M^{+}X^{+}{}_{5'} \qquad X\ N^{+}\ -$ oder

$- X\ M\ ^{3'} \qquad N\ Y^{+}\ -$

$- X^{+}M^{+}{}_{5'} \qquad N^{+}Y\ -$ oder

$- M\ X\ ^{3'} \qquad Y^{+}N\ -$

$- M^{+}X^{+}{}_{5'} \qquad Y\ N^{+}\ -$

wobei

M und N jeweils eine einzige Base aus der Gruppe Adenosin, Cytidin, Guanosin und Tymidin darstellen, M+ und N+ die zu M und N komplementären Basen darstellen, M nicht gleich N ist, X, Y und Z jeweils ein oder mehrere Basen aus der Gruppe Adenosin, Cytidin, Guanosin und Tymidin bedeuten, X+, Y+ und Z+ die zu X, Y und Z komplementären Basen bedeuten, die durch X, Y und Z wiedergegebenen Basen bzw. Basenkombinationen untereinander nicht identisch sind und für Typ (i)

Y nicht gleich Y+ ist.

Bei dem doppelstrangigen Vektor handelt es sich um klonierbare ringförmige DNS.

Vorzugsweise liegen die Insertionsstelle (a) und die Markierungsstelle (b) unmittelbar nebeneinander oder bis zu 1 000, vorzugsweise bis zu 100 und insbesondere bis zu 10 Basenpaare auseinander. Bei der Insertionsstelle (a) kann es sich um eine solche zum Einsetzen von Fremd-DNS mit vorstehenden Enden (protruding ends) oder stumpfen Enden (blunt ends) handeln.

Zur Sequenzierung von Fremd-DNS geht man nun so vor, daß man

— zu sequenzierende Fremd-DNS in die Insertionsstelle (a) des Vektors einbaut,

— den gebildeten Hybridvektor kloniert,

— die Markierungsstelle (b) des klonierten Hybridvektors schneidet,

— die gebildeten Enden des geöffneten Hybridvektors in Gegenwart von DNS-Polymerase und eines oder mehrerer der Triphosphate dGTP, dTTP, dCTP und dATP ergänzt, wobei eines bzw. das eine der Triphosphate (beispielsweise radioaktiv) markiert ist, und dadurch den Hybridvektor einzelmarkiert und

— den markierten geöffneten Hybridvektor in an sich bekannter Weise basenspezifisch abbaut und die Abbauprodukte in an sich bekannter Weise analysiert.

Natürlich kann man in das erfindungsgemäße Verfahren als Fremd-DNS sich überlappende DNS-Fragmente einsetzen, die man durch statistisches Zerkleinern eines größeren DNS-Moleküls gewonnen hat.

Im erfindungsgemäßen Vektor erfüllt die Insertionsstelle (a) eine andere Funktion als die Markierungsstelle (b). Während die Insertionsstelle (a) zu sequenzierende Fremd-DNS aufnehmen soll, soll die Markierungsstelle (b) dazu dienen, den geöffneten Hybridvektor einzelzumarkieren.

Die erfindungsgemäße Einzelmarkierung des Hybridvektors wird durch das folgende Beispiel schematisch näher erläutert.

(Siehe Tabelle Seite 4 f.)

Markierungsstelle

| | | |
|---|---|---|
| - TTTCAAA -<br>- AAAGTTT - | Schneiden | - T<br>- AAAGTT |
| | | TTCAAA -<br>T - |
| Ergänzung mit dGTP, dCTP, dATP | - T<br>- AAAGTT | TTCAAA -<br>T - |
| Ergänzung mit dGTP, dTTP, dC*TP | - TTTC*<br>- AAAGTT | TTCAAA -<br>GTTT - |
| Ergänzung mit dG*TP, dTTP, dCTP | - TTTC<br>- AAAGTT | TTCAAA -<br>G*TTT - |
| Ergänzung mit dGTP, dTTP, dATP | - TTT<br>- AAAGTT | TTCAAA -<br>AAGTTT - |
| Ergänzung mit dTTP, dCTP, dATP | - TTTCAA<br>- AAAGTT | TTCAAA -<br>TTT - |

Anmerkungen :
G = Guanosin, T = Tymidin, C = Cytidin, A = Adenosin ;
* = Markierung

Aus dem gewählten Beispiel ergibt sich, daß der Fachmann aufgrund der ihm bekannten Art der Markierungsstelle vorhersehen kann, mit welchem markierten Triphosphat bzw. mit welcher Kombination von Triphosphaten und mit welchem innerhalb der Kombination markierten Triphosphat er bei der gegebenen Markierungsstelle eine Einzelmarkierung erzielen kann. Im gewählten Fall ist das mit den Triphosphatmischungen dGTP, dTTP und dC*TP oder dG*TP, dTTP und dCTP der Fall.

Gemäß einer weiteren Ausführungsform der Erfindung wird ein doppelstrangiger Vektor mit

— einer im Vektor nur einmal vorkommenden Insertionsstelle (a) für Fremd-DNS,

— einer im Vektor nur einmal vorkommenden einzelmarkierbaren Markierungsstelle (b) beispielsweise des vorstehend angegebenen Typs (i), (ii) oder (iii) und

— einer zusätzlichen im Vektor nur einmal vorkommenden Insertionsstelle (c) für Fremd-DNS vorgesehen,

wobei die eine der beiden Insertionsstellen (a) und (c) beim Schneiden vorstehende Enden und die andere Insertionsstelle stumpfe Enden liefert.

Vorzugsweise liegen die beiden Insertionsstellen (a) und (c) auf derselben Seite der Markierungsstelle (b) in deren Nachbarschaft. Die Insertionsstellen (a) und (c) liegen vorzugsweise unmittelbar nebeneinander oder bis zu 1 000, vorzugsweise bis zu 100 und insbesondere bis zu 10 Basenpaare auseinander.

Ein Beispiel für einen derartigen Vektor stellt pCSVO3 dar, der nachstehend noch näher erläutert wird.

Bei dieser Ausführungsform der Erfindung geht man zur Sequenzierung von Fremd-DNS so vor, daß man

— beide Enden der zu sequenzierenden Fremd-DNS mit einer Insertionsstelle versieht, die einer der beiden Insertionsstellen (a) oder (c) des Vektors entspricht,

— die derart ausgebildete Fremd-DNS unter Bildung von Fragmenten zerkleinert, die gegen das beim Schneiden der Insertionsstellen (a) und (c) des Vektors anfallende DNS-Fragment ausgetauscht werden können,

— die Fremd-DNS gegen das zwischen den beiden Insertionsstellen (a) und (c) des Vektors herausgeschnittene DNS-Fragment austauscht,

— den gebildeten Hybridvektor kloniert,

— die Markierungsstelle (b) des klonierten Hybridvektors schneidet,

— die gebildeten Enden des geöffneten Hybridvektors in Gegenwart von DNS-Polymerase und eines oder mehrerer der Triphosphate dGTP, dTTP, dCTP und dATP ergänzt, wobei eines bzw. das eine der Triphosphate (beispielsweise radioaktiv) markiert ist, und den Hybridvektor einzelmarkiert und

— den markierten geöffneten Hybridvektor in an sich bekannter Weise basenspezifisch abbaut und die Abbauprodukte in an sich bekannter Weise analysiert.

Die Maßnahme, beide Enden der zu sequenzierenden Fremd-DNS mit einer Insertionsstelle zu versehen, die einer Insertionsstelle (a) oder (c) (vorzugsweise für vorstehende Enden) des Vektors entspricht, kann man auch so ausdrücken, daß man die beiden Enden der zu sequenzierenden Fremd-DNS mit einem Linker versieht.

Gemäß einer weiteren Ausführungsform der Erfindung wird ein doppelstrangiger Vektor mit

— einer im Vektor nur einmal vorkommenden Insertionsstelle (a) für Fremd-DNS,

— einer im Vektor nur einmal vorkommenden einzelmarkierbaren Markierungsstelle (b) beispielsweise des vorstehend angegebenen Typs (i), (ii) oder (iii) und

— einer zusätzlichen im Vektor nur einmal vorkommenden Markierungsstelle vom Typ der ersten Markierungsstelle (b), die sich in ihrer Sequenz von dieser unterscheidet.

Vorzugsweise flankieren die beiden Markierungsstellen (b) und (d) die Insertionsstelle (a) und sind in ihrer Nachbarschaft angeordnet. Vorzugsweise liegen die beiden Markierungsstellen (b) und (d) unmittelbar neben der Insertionsstelle (a) oder bis zu 1 000, vorzugsweise bis zu 100 und insbesondere bis zu 10 Basenpaare von ihr entfernt.

Wie bereits gesagt, sollen beide Markierungsstellen (b) und (d) nur einmal im Vektor vorkommen. Es ist jedoch vorteilhaft, wenn sie von demselben Restriktionsenzym geschnitten werden können. Bei der Insertionsstelle (a) kann es sich um eine solche zum Einsetzen von Fremd-DNS mit vorstehenden Enden oder stumpfen Enden handeln.

Zur Sequenzierung von Fremd-DNS mit einem Vektor dieser Ausführungsform geht man nun so vor, daß man

— zu sequenzierende Fremd-DNS in die Insertionsstelle (a) des Vektors einbaut,

— den gebildeten Hybridvektor kloniert,

— die beiden Markierungsstellen (b) und (d) des klonierten Hybridvektors schneidet,

— die beiden Enden des Schnittfragmentes mit der eingebauten Fremd-DNS in Gegenwart von DNS-Polymerase und eines oder mehrerer der Triphosphate dGTP, dTTP, dCTP und dATP ergänzt, wobei eines bzw. das eine der Triphosphate markiert ist, und einmal nur das eine Ende des Schnittfragmentes und einmal nur dessen anderes Ende einzelmarkiert und

— das markierte Schnittfragment in an sich bekannter Weise basenspezifisch abbaut und die Abbauprodukte in an sich bekannter Weise analysiert.

Die erfindungsgemäße Einzelmarkierung des Schnittfragmentes mit der eingebauten Fremd-DNS wird durch das folgende Beispiel schematisch näher erläutert.

```
GCGAC -              - GTCG
         Fremd-DNS
 GCTG -              - CAGCT

Ergänzung mit
 *
dATP, dGTP                                          *
------------->  GCGAC -              - GTCGA
                          Fremd-DNS
                 GCTG -              - CAGCT

Ergänzung mit
 *
dCTP, dGTP      GCGAC -              - GTCG
                *         Fremd-DNS
------------->  CGCTG -              - CAGCT
```

Aus dem gewählten Beispiel ergibt sich, daß der Fachmann aufgrund der ihm bekannten Art der Markierungsstellen (b) und (d) vorhersehen kann, mit welchem markierten Triphosphat bzw. mit welcher Kombination von Triphosphaten und mit welchem innerhalb der Kombination markierten Triphosphat er bei einer der beiden Markierungsstellen eine Einzelmarkierung erzielen kann. Das Beispiel zeigt, daß man entweder die eine oder die andere Markierungsstelle einzelmarkieren kann. Dementsprechend kann man bei dieser Ausführungsform der Erfindung beide Stränge der Fremd-DNS sequenzieren. Dabei kann man die beiden Stränge vollständig sequenzieren, wodurch man die Sicherheit der Sequenzanalyse erhöhen kann. Man kann jedoch die beiden Stränge auch nur bis zu einem ihnen gemeinsamen Überlappungsbereich sequenzieren ; dieses Vorgehen ist besonders bei der Sequenzierung von sehr langen Fremd-DNS-Strängen vorteilhaft.

Zur Herstellung der erfindungsgemäßen Vektoren muß der Fachmann DNS-Moleküle sequenzeiren, schneiden und verknüpfen können. Auf die dem Fachmann zur Verfügung stehenden Sequenzierungsmethoden wurde bereits einleitend eingegangen. Zum Schneiden und Verknüpfen von DNS-Molekülen sei auf das deutsche Patent P 28 14 039.8 und die darin zitierte Literatur hingewiesen. Sowohl die Insertionsstellen (a) und (c) als auch die Markierungsstellen (b) und (d) der erfindungsgemäßen Vektoren können durch Restriktionsendonukleasen bzw. Restriktionsenzyme geschnitten werden. Der Fachmann ist auch mit dem statistischen Zerkleinern größerer DNS-Moleküle zu kleineren Fragmenten vertraut, der

physikalisch (z. B. mit Ultraschall) oder chemisch (z. B. enzymatisch) erfolgen kann.

Erfindungsgemäß wird also eine Reihe neuer Sequenzierungsvektoren vorgesehen, die die Anzahl der Herstellungsstufen vor den basenspezifischen Abbaureaktionen auf eine einzige Restriktion und eine Auffüll-Markierungsreaktion vereinfachen. Nach dieser Methode wird kein überlappender unlesbarer Bereich gebildet. Ferner führt das erfindungsgemäße Subklonierungsprotokoll praktisch zu keinen Hintergrund- oder Untergrundklonen. Ein Prototyp für geeignete Sequenzierungsplasmide wird nachstehend näher erläutert und mit pCSVO3 bezeichnet.

Herstellung von pCSV03

pGV001 ist ein 1882bp-Derivat von pBR327 ; es wird durch Spalten von pBR327 mit HindIII und AvaI, Auffüllen der vorstehenden Enden und Selbstverknüpfung der stumpfen Enden gebildet. pGV001 trägt einen Replikationsursprung und das β-Laktamasegen. Die Einzel-EcoRI-, -ClaI- und -HindIII-Stellen liegen hinter diesen Bereichen. pCSV03 wurde durch Einführen eines chemisch synthetisierten DNS-Fragments (10 bp ds) in die aufgefüllte ClaI-Stelle von pGV001 gebildet.

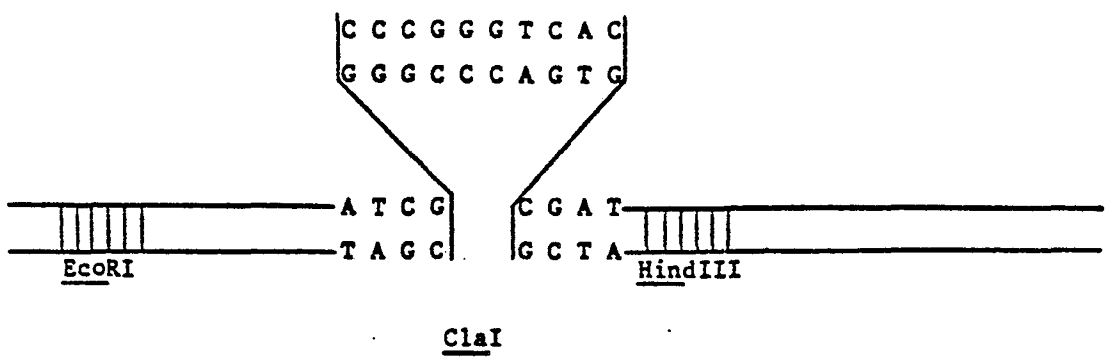


pCSV03 enthält Einzelspaltstellen für EcoRI, SmaI, EcaI (= BstEII) und HindIII.

Eine Spaltung mit EcaI (= BstEII) liefert ein linearisiertes Vektormolekül mit zwei unterschiedlichen vorstehenden 5'-Enden.

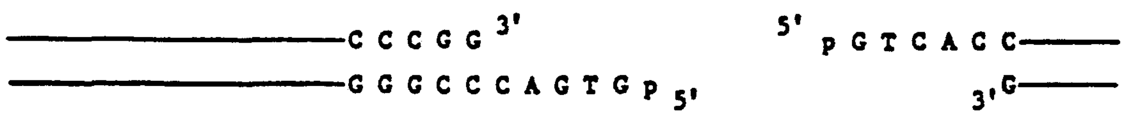


Eine Auffüllreaktion in Gegenwart von dGTP, dTTP und alpha $^{32}$P-dCTP markiert nur das linke Ende. Da dATP in der Reaktionsmischung fehlt, kann das rechte Ende nicht markiert werden.

pCSV03 zeigt für eine Subklonierungs-Sequenzierung folgende Merkmale :

(1) Eine EcaI- bzw. BstEII-Spaltstelle, die mit einer einzigen Auffüllreaktion einzelmarkiert werden kann.

(2) Eine SmaI-Spaltstelle, die die EcaI-Stelle überlappt. SmaI kann als Insertionsstelle mit stumpfem Ende verwendet werden. Jedes Fragment, das an dieser Stelle eingeführt wird, grenzt an EcaI an, so daß es leicht auf dieser Seite einzelmarkiert werden kann.

(3) EcoRI-Stelle 23bp entfernt von SmaI-Stelle, entgegengesetzt zu EcaI.

Der Vektor kann zweifach mit EcoRI und SmaI gespalten und für orientierendes Subklonieren von nach Größe fraktionierten DNS-Fragmenten verwendet werden und ein progressives Sequenzieren ermöglichen ; vgl. wie folgt.

(4) Ein kleiner (1894bp) phosphatasebehandelter und mit SmaI-linearisierter Vektor kann leicht präparativ auf einem niedrigschmelzenden Agarosegel (2 %) gereinigt werden. Ein auf diese Weise gereinigter Vektor liefert weniger als 1 % Untergrundklone infolge Eigenverknüpfung.

(5) Die Transformationsselektion beruht auf dem Ampicillinresistenzgen.

Subklonieren in pCSV03

Statistisches Subklonieren (Shotgun-Subklonieren) :

Fragmente mit stumpfem Ende aus Restriktionen, Hydrolysat mit DNase oder beschallte DNS.

Mit SmaI gespaltener dephosphorylierter Vektor.

Ligationsreaktion

Transformation.

Orientiertes Subklonieren :

Verknüpfen von EcoRI-Linkern mit dem zu sequenzierenden DNS-Molekül
Beschallen von DNS oder Behandeln mit DNase
Spalten mit EcoRI
Fraktionieren auf einem niedrigschmelzenden Agarosegel (2 %)
Verknüpfen von Größenfraktionen mit einem mit EcoRI-SmaI behandelten pCSV03
Transformieren einzelner Fraktionen.

Nur Fragmente mit einem ursprünglichen Ende können mit dem Vektor verknüpft werden, da sie allein ein klebriges EcoRI-Ende an einer Seite tragen. Die entgegengesetzte Seite (das stumpfe Ende) grenzt an EcaI an. Daher bilden verschiedene Gelfraktionen eine Gruppe überlappender Sequenzen.

Ursprüngliches DNS-Fragment :
In Rekombinationssubklonen
enthaltenes Fragment

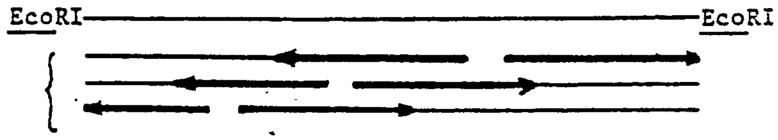


Dicke Pfeile geben nacheinander sequenzierte Segmente wieder. Anmerkung : Ein DNS-Molekül, das ein oder mehrere EcoRI-Stellen enthält, kann ohne Zugabe von EcoRI-Linkern verwendet werden. Das Molekül wird vor seiner Beschallung zirkularisiert. Eine EcoRI-Spaltung muß der Beschallung folgen.

Sequenzieren von Rekombinationssubklonen von pCSV03

1. Man picke Kolonien heraus und lasse über Nacht 1-ml-Kulturen wachsen.
2. Man zentrifugiere die Zellen, lysiere (z. B. gemäß der Erhitzungsarbeitsweise von Quigley und Holmes) und kläre.
3. Man fälle die Rekombinations-DNSen.
4. Man spalte mit BstEII, einem im Handel erhältlichen Enzym.
5. Man fülle mit Klenow-Polymerase und dGTP, dTTP und alpha$^{32}$P-dCTP auf.
6. Man zentrifugiere rasch durch Sephadex G50 (1 ml).
7. Man leite chemische Abbaureaktionen ein.

Herstellung von pCSV31

Zur Herstellung von pCSV31 wird pCSV03 mit dem Restriktionsenzym SmaI geschnitten. In die Schnittstelle wird ein chemischsynthetisiertes doppelstrangiges DNS-Fragment (20 bp) folgender Sequenz eingesetzt :

TGACTAAGTCGACTCAGTCA
ACTGATTCAGCTGAGTCAGT

Die beiden Markierungsstellen (b) und (d) unterscheiden sich durch unterschiedliche überstehende Ende. pCSV31 ist also durch folgende Merkmale gekennzeichnet :

(1) eine SalI-Spaltstelle als erste Insertionsstelle (a).
(2) Eine Tth111I-Spaltstelle als Markierungsstelle (b).
(3) Eine Tth111I-Spaltstelle als Markierungsstelle (d),
(4) wobei sich (b) und (d) in ihrer Sequenz unterscheiden.
(5) Eine EcoRI-Spaltstelle als Insertionsstelle (c).
(6) Das Ampicillinresistenzgen.
(7) Eine Länge von weniger als 2 000 Basenpaaren.

pBR327 hinterlegt am 22 April 1983 als DSM 2629 ; Gene, 9 (1980) 287-305
bp Basenpaar(e)
ds doppelsträngig
dGTP Desoxyguanosintriphosphat
dTTP Desoxytymidintriphosphat
dCTP Desoxycytidintriphosphat
dATP Desoxyadenosintriphosphat

Im ganzen Text soll der Ausdruck « Base » für A (Adenosin), C (Cytidin), G (Guanosin) und T (Thymidin) heißen : « Nukleosid ».

**Patentansprüche**

1. Doppelstrangiger Sequenzierungsvektor mit

a) einer im Vektor nur einmal vorkommenden Insertionsstelle für Fremd-DNS und

b) einer im Vektor nur einmal vorkommenden Markierungsstelle des folgenden Typs, die einzelmarkiert werden kann :

(i)

$- X^{3'} \quad YX^{+} -$

$- X^{+} \; Y^{+}{}_{5'} \quad X -$ oder

$- X^{3'} \quad YZ -$

$- X^{+} \; Y^{+}{}_{5'} \quad Z^{+} -$ oder

(ii)

$- X\,M \quad Z^{3'} \quad N^{+}X^{+} -$

$- X^{+}M^{+}{}_{5'} \quad Z^{+}N\,X -$ oder

$- X\,M \quad Z^{3'} \quad N^{+}Y -$

$- X^{+}M^{+}{}_{5'} \quad Z^{+}N\,Y^{+} -$ oder

(iii)

$- X\,M^{3'} \quad N\,X^{+} -$

$- X^{+}M^{+}{}_{5'} \quad N^{+}X -$ oder

$- M\,X^{3'} \quad X^{+}N -$

$- M^{+}X^{+}{}_{5'} \quad X\,N^{+} -$ oder

$- X\,M^{3'} \quad N\,Y^{+} -$

$- X^{+}M^{+}{}_{5'} \quad N^{+}Y -$ oder

$- M\,X^{3'} \quad Y^{+}N -$

$- M^{+}X^{+}{}_{5'} \quad Y\,N^{+} -$

wobei

M und N jeweils eine einzige Base aus der Gruppe Adenosin, Cytidin, Guanosin und Tymidin darstellen, $M^{+}$ und $N^{+}$ die zu M und N komplementären Basen darstellen, M nicht gleich N ist, X, Y und Z jeweils ein oder mehrere Basen aus der Gruppe Adenosin, Cytidin, Guanosin und Tymidin bedeuten, $X^{+}$, $Y^{+}$ und $Z^{+}$ die zu X, Y und Z komplementären Basen bedeuten, die durch X, Y und Z wiedergegebenen Basen bzw. Basenkombinationen untereinander nicht identisch sind und für Typ (i)

Y nicht gleich Y+ ist.

2. Sequenzierungsvektor nach Anspruch 1, dadurch gekennzeichnet, daß die Insertionsstelle (a) und die Markierungsstelle (b) unmittelbar nebeneinander oder bis zu 1 000, vorzugsweise bis zu 100 und insbesondere bis zu 10 Basenpaare auseinander liegen.

3. Sequenzierungsvektor nach einem der Ansprüche 1 bis 2, dadurch gekennzeichnet, daß es sich bei der Insertionsstelle (a) um eine solche zum Einsetzen von Fremd-DNS mit vorstehenden Enden oder stumpfen Enden handelt.

4. Sequenzierungsvektor nach einem der Ansprüche 1 bis 3, gekennzeichnet durch (c) eine zusätzliche Insertionsstelle für Fremd-DNS, wobei die eine der beiden Insertionsstellen (a) und (c) beim Schneiden vorstehende Enden und die andere Insertionsstelle stumpfe Enden liefert.

5. Sequenzierungsvektor nach Anspruch 4, dadurch gekennzeichnet, daß die beiden Insertionsstellen (a) und (c) auf derselben Seite der Markierungsstelle (b) in deren Nachbarschaft liegen, wobei die beim Schneiden stumpfe Enden liefernde Insertionsstelle vorzugsweise näher an der Markierungsstelle (b) liegt.

6. Sequenzierungsvektor nach Anspruch 4 oder 5, dadurch gekennzeichnet, daß die beiden Insertionsstellen (a) und (c) unmittelbar nebeneinander oder bis zu 1 000, vorzugsweise bis zu 100 und insbesondere bis zu 10 Basenpaare auseinander liegen.

7. Sequenzierungsvektor nach einem der Ansprüche 4 bis 6, gekennzeichnet durch folgende Merkmale :

— eine SmaI-Spaltstelle als erste Insertionsstelle (a)

— eine EcaI- bzw. BstEII-Spaltstelle als Markierungsstelle (b) ;

— eine EcoRI-Spaltstelle als Insertionsstelle (c) ;

— das Ampicillinresistenzgen ; und

— eine Länge von weniger als 2 000 Basenpaaren.

8. Sequenzierungsvektor nach einem der Ansprüche 1 bis 3, gekennzeichnet durch (d) eine zusätzliche im Sequenzierungsvektor nur einmal vorkommende Markierungsstelle vom Typ der ersten Markierungsstelle (b), die sich in ihrer Sequenz von der Markierungsstelle (b) unterscheidet.

9. Sequenzierungsvektor nach Anspruch 8, dadurch gekennzeichnet, daß die beiden Markierungsstellen (b) und (d) die Insertionsstelle (a) flankieren und in ihrer Nachbarschaft angeordnet sind.

10. Sequenzierungsvektor nach Anspruch 8 oder 9, dadurch gekennzeichnet, daß die beiden Markierungsstellen (b) und (d) unmittelbar neben der Insertionsstelle (a) oder bis zu 1 000, vorzugsweise bis zu 100 und insbesondere bis zu 10 Basenpaare von ihr entfernt liegen.

11. Sequenzierungsvektor nach einem der Ansprüche 8 bis 10, gekennzeichnet durch folgende Merkmale :

— eine SalI-Spaltstelle als erste Insertionsstelle (a),

— eine Tth111I-Spaltstelle als Markierungsstelle (b),

— eine Tth111I-Spaltstelle als Markierungsstelle (d),

— wobei sich (b) und (d) in ihrer Sequenz unterscheiden,

— eine EcoRI-Spaltstelle als Insertionsstelle (c),

— das Ampicillinresistenzgen und

— eine Länge von weniger als 2 000 Basenpaaren.

12. Verfahren zur Sequenzierung von Fremd-DNS, dadurch gekennzeichnet, daß man

— zu sequenzierende Fremd-DNS in die Insertionsstelle (a) eines Sequenzierungsvektors gemäß einem der Ansprüche 1 bis 3 einbaut,

— den gebildeten Hybridvektor kloniert,

— die Markierungsstelle (b) des klonierten Hybridvektors schneidet.

— die gebildeten Enden des geöffneten Hybridvektors in Gegenwart von DNS-Polymerase und eines oder mehrerer der Triphosphate dGTP, dTTP, dCTP und dATP ergänzt, wobei eines der Triphosphate (beispielsweise radioaktiv) markiert ist, und den Hybridvektor einzelmarkiert und

— den markierten geöffneten Hybridvektor in an sich bekannter Weise basenspezifisch abbaut und die Abbauprodukte in an sich bekannter Weise analysiert.

13. Verfahren nach Anspruch 12, dadurch gekennzeichnet, daß man in das Verfahren als Fremd-DNS sich überlappende DNS-Fragmente einsetzt, die man durch statistisches Zerkleinern eines größeren DNS-Moleküls gewonnen hat.

14. Verfahren zur Sequenzierung von Fremd-DNS, dadurch gekennzeichnet, daß man

— beide Enden der zu sequenzierenden Fremd-DNS mit einer Insertionsstelle versieht, die einer der beiden Insertionsstellen (a) oder (c) eines Sequenzierungsvektors gemäß einem der Ansprüche 4 bis 7 entspricht,

— die derart ausgebildete Fremd-DNS unter Bildung von Fragmenten zerkleinert, die gegen das beim Schneiden der Insertionsstellen (a) und (c) des Sequenzierungsvektors anfallende DNS-Fragment ausgetauscht werden können,

— die Fremd-DNS gegen das zwischen den beiden Insertionsstellen (a) und (c) des Sequenzierungsvektors herausgeschnittene DNS-Fragment austauscht,

— den gebildeten Hybridvektor kloniert,

— die Markierungsstelle (b) des klonierten Hybridvektors schneidet,

— die gebildeten Enden des geöffneten Hybridvektors in Gegenwart von DNS-Polymerase und eines oder mehrerer der Triphosphate dGTP, dTTP, dCTP und dATP ergänzt, wobei eines der Triphosphate (beispielsweise radioaktiv) markiert ist, und den Hybridvektor einzelmarkiert und

— den markierten geöffneten Hybridvektor in an sich bekannter Weise basenspezifisch abbaut und die Abbauprodukte in an sich bekannter Weise analysiert.

15. Verfahren nach Anspruch 14, dadurch gekennzeichnet, daß man

— beide Enden der zu sequenzierenden Fremd-DNS mit einer Insertionsstelle versieht, die einer der

beiden Insertionsstellen (a) oder (c) für vorstehende Enden eines Sequenzierungsvektors gemäß einem der Ansprüche 4 bis 7 entspricht, und

— die derart ausgebildete Fremd-DNS unter Bildung von Fragmenten mit einem stumpfen Ende und einem die Insertionsstelle tragenden Ende abbaut und die Insertionsstelle (vor oder nach dem Zerkleinern) spaltet.

16. Verfahren nach Anspruch 14 oder 15, dadurch gekennzeichnet, daß man mit einem Selektionsmarker (vorzugsweise in Nachbarschaft zur Insertionsstelle der Fremd-DNS) versehene Fremd-DNS verwendet.

17. Verfahren zur Sequenzierung von Fremd-DNS, dadurch gekennzeichnet, daß man

— zu sequenzierende Fremd-DNS in die Insertionsstelle (a) eines Sequenzierungsvektors gemäß einem der Ansprüche 8 bis 10 einbaut,

— den gebildeten Hybridvektor kloniert,

— die beiden Markierungsstellen (b) und (d) des klonierten Hybridvektors schneidet,

— die beiden Enden des Schnittfragmentes mit der eingebauten Fremd-DNS in Gegenwart von DNS-Polymerase und eines oder mehrerer der Triphosphate dGTP, dTTP, dCTP und dATP ergänzt, wobei eines der Triphosphate (beispielsweise radioaktiv) markiert ist, und einmal nur das eine Ende des Schnittfragmentes und einmal nur dessen anderes Ende einzelmarkiert, und

— das markierte Schnittfragment in an sich bekannter Weise basenspezifisch abbaut und die Abbauprodukte in an sich bekannter Weise analysiert.

**Claims**

1. Double-strand sequencing vector having

a) an insertion site for foreign DNA that occurs only once in the vector, and

b) a labelling site of the following type that occurs only once in the vector and can be individually labelled :

(i) $- X\ {}^{3'} \qquad YX^{+} -$

$- X^{+}\ Y^{+}{}_{5'} \qquad X -$ or

$- X\ {}^{3'} \qquad YZ -$

$- X^{+}\ Y^{+}{}_{5'} \qquad Z^{+} -$ or

(ii) $- X\ M\ \ Z^{3'} \qquad N^{+}X^{+} -$

$- X^{+}M^{+}{}_{5'} \qquad Z^{+}N\ X -$ or

$- X\ M\ \ Z^{3'} \qquad N^{+}Y -$

$- X^{+}M^{+}{}_{5'} \qquad Z^{+}N\ Y^{+} -$ or

(iii) $- X\ M\ {}^{3'} \qquad N\ X^{+} -$

$- X^{+}M^{+}{}_{5'} \qquad N^{+}X -$ or

$- M\ X\ {}^{3'} \qquad X^{+}N -$

$- M^{+}X^{+}{}_{5'} \qquad X\ N^{+} -$ or

$$
\begin{array}{ll}
- X\ M\ ^{3'} & N\ Y^{+}\ - \\
- X^{+}M^{+}{}_{5'} & N^{+}Y\ - \text{ or} \\
- M\ X\ ^{3'} & Y^{+}N\ - \\
- M^{+}X^{+}{}_{5'} & Y\ N^{+}\ -
\end{array}
$$

in which

M and N each represent a single base from the group comprising adenosine, cytidine, guanosine and thymidine, M+ and N+ represent the bases complementary to M and N, M is not the same as N, X, Y and Z each represent one or more bases from the group comprising adenosine, cytidine, guanosine and thymidine, X+, Y+ and Z+ represent the bases complementary to X, Y and Z, the bases or combinations of bases represented by X, Y and Z are not identical to one another, and, for type (i),

Y is not the same as Y+.

2. Sequencing vector according to claim 1, characterised in that the insertion site (a) and the labelling site (b) are directly adjacent to one another or are up to 1 000, preferably up to 100 and especially up to 10, base pairs apart.

3. Sequencing vector according to claim 1 or 2, characterised in that the insertion site (a) is a site for the insertion of foreign DNA having projecting ends or blunt ends.

4. Sequencing vector according to any one of claims 1 to 3, characterised by (c) an additional insertion site for foreign DNA, one of the two insertion sites (a) and (c) producing projecting ends when cut and the other insertion site producing blunt ends.

5. Sequencing vector according to claim 4, characterised in that the two insertion sites (a) and (c) are located on the same side of the labelling site (b) and in the vicinity thereof, the insertion site that produces blunt ends when cut preferably being closer to the labelling site (b).

6. Sequencing vector according to claim 4 or 5, characterised in that the two insertion sites (a) and (c) are directly adjacent to one another or are up to 1 000, preferably up to 100 and especially up to 10, base pairs apart.

7. Sequencing vector according to any one of claims 4 to 6, characterised by the following features :

— an SmaI-cleavage site as the first insertion site (a) ;

— an EcaI- or BstEII-cleavage site as the labelling site (b) ;

— an EcoRI-cleavage site as insertion site (c) ;

— the ampicillin resistance gene ; and

— a length of less than 2 000 base pairs.

8. Sequencing vector according to any one of claims 1 to 3, characterised by (d) an additional labelling site that occurs only once in the sequencing vector and is of the same type as the first labelling site (b) but differs from labelling site (b) in its sequence.

9. Sequencing vector according to claim 8, characterised in that the two labelling sites (b) and (d) flank the insertion site (a) and are arranged in the vicinity thereof.

10. Sequencing vector according to claim 8 or 9, characterised in that the two labelling sites (b) and (d) are located directly adjacent to the insertion site (a) or are up to 1 000, preferably up to 100 and especially up to 10, base pairs away from the insertion site (a).

11. Sequencing vector according to any one of claims 8 to 10, characterised by the following features :

— an SalI-cleavage site as the first insertion site (a) ;

— a Tth111I-cleavage site as labelling site (b),

— a Tth111I-cleavage site as labelling site (d), (b) and (d) being different in sequence,

— an EcoRI-cleavage site as insertion site (c),

— the ampicillin resistance gene, and

— a length of less than 2 000 base pairs.

12. Process for the sequencing of foreign DNA, characterised in that

— the foreign DNA to be sequenced is incorporated into insertion site (a) of a sequencing vector according to any one of claims 1 to 3,

— the resulting hybrid vector is cloned,

— the labelling site (b) of the cloned hybrid vector is cut,

— the resulting ends of the opened hybrid vector are supplemented in the presence of DNA-polymerase and one or more of the triphosphates dGTP, dTTP, dCTP and dATP, one of the triphosphates being labelled (for example radioactively), and the hybrid vector is individually labelled, and the labelled opened hybrid vector is degraded basespecifically in a manner known *per se* and the degradation products are analysed in a manner known *per se*.

13. Process according to claim 12, characterised in that overlapping DNA fragments that have been

obtained by statistical reduction of a larger DNA molecule are used as the foreign DNA in the process.

14. Process for the sequencing of foreign DNA, characterised in that

— both ends of the foreign DNA to be sequenced are provided with an insertion site that corresponds to one of the two insertion sites (a) or (c) of a sequencing vector according to any one of claims 4 to 7,

— the foreign DNA constructed in that manner is reduced to form fragments that can be substituted for the DNA fragment formed when the insertion sites (a) and (c) of the sequencing vector are cut,

— the foreign DNA is substituted for the DNA fragment cut out between the two insertion sites (a) and (c) of the sequencing vector,

— the resulting hybrid vector is cloned,

— the labelling site (b) of the cloned hybrid vector is cut,

— the resulting ends of the opened hybrid vector are supplemented in the presence of DNA-polymerase and one or more of the triphosphates dGTP, dTTP, dCTP and dATP, one of the triphosphates being labelled (for example radioactively), and the hybrid vector is individually labelled, and

— the labelled opened hybrid vector is degraded basespecifically in a manner known *per se* and the degradation products are analysed in a manner known *per se.*

15. Process according to claim 14, characterised in that

— both ends of the foreign DNA to be sequenced are provided with an insertion site that corresponds to one of the two insertion sites (a) or (c) for projecting ends of a sequencing vector according to any one of claims 4 to 7, and

— the foreign DNA constructed in that manner is degraded to form fragments having a blunt end and an end carrying the insertion site, and the insertion site is cleaved (before or after reduction).

16. Process according to claim 14 or 15, characterised in that foreign DNA provided with a selection marker (preferably in the vicinity of the insertion site of the foreign DNA) is used.

17. Process for the sequencing of foreign DNA, characterised in that

— the foreign DNA to be sequenced is incorporated into the insertion site (a) of a sequencing vector according to any one of claims 8 to 10,

— the resulting hybrid vector is cloned,

— the two labelling sites (b) and (d) of the cloned hybrid vector are cut,

— the two ends of the cut fragment having the incorporated foreign DNA are supplemented in the presence of DNA-polymerase and one or more of the triphosphates dGTP, dTTP, dCTP and dATP, one of the triphosphates being labelled (for example radioactively), and in one case only one end of the cut fragment and in another case only its other end is individually labelled, and

— the labelled cut fragment is degraded base-specifically in a manner known *per se* and the degradation products are analysed in a manner known *per se.*

**Revendications**

1. Vecteur à double brin pour séquençage, comportant :

a) un site d'insertion d'ADN étranger, n'apparaissant qu'une seule fois dans le vecteur, et

b) un site de marquage, n'apparaissant qu'une seule fois dans le vecteur, site de types suivants pouvant être soumis à marquage unique (sur un seul brin) :

(i) $- X\ {}^{3'} \qquad YX^{+} -$

$- X^{+}\ Y^{+}{}_{5'} \qquad X -$ ou

$- X\ {}^{3'} \qquad YZ -$

$- X^{+}\ Y^{+}{}_{5'} \qquad Z^{+} -$ ou

(ii) $- X\ M\ \ Z^{3'} \qquad N^{+}X^{+} -$

$- X^{+}M^{+}{}_{5'} \qquad Z^{+}N\ X -$ ou

$- X\ M\ \ Z^{3'} \qquad N^{+}Y -$

$- X^{+}M^{+}{}_{5'} \qquad Z^{+}N\ Y^{+} -$ ou

(iii)
$$\begin{array}{ll} -X\ M^{3'} & N\ X^{+}\ - \\ -X^{+}M^{+}{}_{5'} & N^{+}X\ - \end{array}$$
ou

$$\begin{array}{ll} -M\ X^{3'} & X^{+}N\ - \\ -M^{+}X^{+}{}_{5'} & X\ N^{+}\ - \end{array}$$
ou

$$\begin{array}{ll} -X\ M^{3'} & N\ Y^{+}\ - \\ -X^{+}M^{+}{}_{5'} & N^{+}Y\ - \end{array}$$
ou

$$\begin{array}{ll} -M\ X^{3'} & Y^{+}N\ - \\ -M^{+}X^{+}{}_{5'} & Y\ N^{+}\ - \end{array}$$

où

M et N représentent à chaque fois une base individuelle de l'ensemble adénosine, cytidine, guanosine et thymidine, M+ et N+ représentent des bases complémentaires de M et N, M n'est pas identique à N, X, Y et Z désignent chaque fois une ou plusieurs bases de l'ensemble adénosine, cytidine, guanosine et thymidine, X+, Y+ et Z+ désignent les bases complémentaires de X, Y et Z, où les bases ou combinaisons des bases représentées par X, Y et Z ne sont pas identiques entre elles, et pour le type (i)

Y n'est pas identique à Y+.

2. Vecteur pour séquençage selon la revendication 1, caractérisé en ce que le site (a) d'insertion et le site (b) de marquage sont immédiatement voisins ou ne sont séparés que par un ensemble allant jusqu'à 1 000, avantageusement jusqu'à 100 et en particulier jusqu'à 10, paires de bases.

3. Vecteur pour séquençage selon l'une des revendications 1 et 2, caractérisé en ce qu'il s'agit, pour le site (a) d'insertion, d'un site pour l'insertion d'un ADN étranger comportant des extrémités saillantes ou des extrémités émoussées ou en retrait.

4. Vecteur pour séquençage selon l'une des revendications 1 à 3, caractérisé en ce qu'il comporte (c) un site supplémentaire d'insertion d'ADN étranger, de sorte qu'au découpage l'un des deux sites (a) et (c) d'insertion fournit des extrémités saillantes et l'autre site d'insertion des extrémités en retrait.

5. Vecteur pour séquençage selon la revendication 4, caractérisé en ce que les deux sites (a) et (c) d'insertion se trouvent du même côté du site (b) de marquage et au voisinage de celui-ci, le site d'insertion fournissant lors du découpage des extrémités en retrait étant avantageusement plus proche du site (b) de marquage.

6. Vecteur pour séquençage selon la revendication 4 ou 5, caractérisé en ce que les deux sites d'insertion (a) et (c) sont immédiatement voisins l'un de l'autre ou ne sont séparés que par un ensemble allant jusqu'à 1 000, avantageusement jusqu'à 100 et en particulier jusqu'à 10 paires de bases.

7. Vecteur pour séquençage selon l'une des revendications 4 à 6, caractérisé par les particularités suivantes :

— un site de coupure par SmaI comme site d'insertion (a)
— un site de coupure par EcaI ou BstEII comme site (b) de marquage ;
— un site de coupure par EcoRI comme site (c) d'insertion ;
— le gène de résistance à l'ampicilline ; et
— une longueur de moins de 2 000 paires de bases.

8. Vecteur pour séquençage selon l'une des revendications 1 à 3, caractérisé en ce qu'il comporte (d) un site supplémentaire de marquage, n'apparaissant qu'une fois dans le vecteur pour séquençage, du type du premier site (b) de marquage qui diffère du site (b) de marquage par sa séquence.

9. Vecteur pour séquençage selon la revendication 8, caractérisé en ce que les deux sites (b) et (d) de marquage entourent ou flanquent le site (a) d'insertion et sont placés au voisinage de celui-ci.

10. Vecteur pour séquençage selon la revendication 8 ou 9, caractérisé en ce que les deux sites (b) et (d) de marquage sont immédiatement voisins du site (a) d'insertion ou n'en sont éloignés que par un ensemble allant jusqu'à 1 000, avantageusement jusqu'à 100 et en particulier jusqu'à 10 paires de bases.

11. Vecteur pour séquençage selon l'une des revendications 8 à 10, caractérisé par les particularités

suivantes :

— un site de coupure par SalI comme premier site (a) d'insertion,

— un site de coupure par Tth111I comme site de marquage (b),

— un site de coupure par Tth111I comme site de marquage (d),

— (b) et (d) étant différents par leurs séquences,

— un site de coupure par EcoRI comme site (c) d'insertion,

— le gène de résistance à l'ampicilline, et

— une longueur inférieure à 2 000 paires de bases.

12. Procédé pour séquencer de l'ADN étranger, caractérisé en ce qu'on incorpore l'ADN étranger à séquencer au site d'insertion (a) d'un vecteur pour séquençage selon l'une des revendications 1 à 3,

— on clone le vecteur hybride formé,

— on découpe le site (b) de marquage du vecteur hybride cloné,

— on allonge les extrémités formées du vecteur hybride ouvert en présence de ADN-polymérase et d'un ou plusieurs des triphosphates dGTP, dTTP, dCTP et dATP, en marquant (par exemple radioactivement) l'un des triphosphates, et l'on effectue un marquage unique sur le vecteur hybride, et

— on dégrade, de façon connue en soi, et de manière spécifique aux bases, le vecteur hybride ouvert et marqué et l'on analyse de façon connue les produits de dégradation.

13. Procédé selon la revendication 12, caractérisé en ce que l'on utilise, comme ADN étranger, des fragments de ADN qui se chevauchent, que l'on a obtenus par fragmentation statistique d'une plus grosse molécule d'ADN.

14. Procédé pour séquencer de l'ADN étranger, caractérisé en ce que :

— on munit les deux extrémités de l'ADN étranger à séquencer d'un site d'insertion correspondant à l'un des deux sites (a) ou (c) d'insertion d'un vecteur pour séquençage selon l'une des revendications 4 à 7,

— on fragmente l'ADN étranger ainsi formé, en formant des fragments que l'on peut remplacer par le fragment de l'ADN obtenu lors du découpage des sites (a) et (c) d'insertion du vecteur pour séquençage,

— on remplace, par l'ADN étranger, le fragment de ADN découpé entre les deux sites (a) et (c) d'insertion du vecteur pour séquençage,

— on clone le vecteur hybride formé,

— on découpe les sites (b) de marquage du vecteur hybride cloné,

— on allonge les extrémités formées du vecteur hybride ouvert en présence de ADN-polymérase et d'un ou plusieurs des triphosphates dGTP, dTTP, dCTP et dATP, de sorte que l'un des triphosphates est marqué (par exemple par voie radioactive), et le vecteur hybride subit un marquage unique, et

— on dégrade, de façon spécifique aux bases et de manière connue en soi, le vecteur hybride ouvert et marqué, et l'on analyse de façon connue en soi les produits de dégradation.

15. Procédé selon la revendication 14, caractérisé en ce que :

— on munit les deux extrémités de l'ADN étranger à séquencer d'un site d'insertion qui correspond à l'un des deux sites (a) ou (c) d'insertion des extrémités saillantes d'un vecteur pour séquençage selon l'une des revendications 4 à 7, et

— on dégrade l'ADN étranger ainsi formé, en formant des fragments comportant une extrémité émoussée et une extrémité portant le site d'insertion, et l'on scinde (avant ou après la fragmentation), le site d'insertion.

16. Procédé selon la revendication 14 ou 15, caractérisé en ce qu'on utilise un ADN étranger muni d'un marqueur de sélection (avantageusement placé au voisinage du site d'insertion de l'ADN étranger).

17. Procédé pour séquencer de l'ADN étranger, caractérisé en ce que :

— on incorpore l'ADN étranger à séquencer au site (a) d'insertion d'un vecteur pour séquençage selon l'une des revendications 8 à 10,

— on clone le vecteur hybride formé,

— on découpe les deux sites (b) et (d) de marquage du vecteur hybride cloné,

— on allonge les deux extrémités du fragment de découpage par l'ADN étranger incorporé en présence de ADN-polymérase et d'un ou plusieurs des triphosphates dGTP, dTTP, dCTP et dATP, l'un des triphosphates étant marqué (par exemple par voie radioactive) et l'on soumet à marquage unique une fois seulement une extrémité du fragment découpé et une fois seulement son autre extrémité, et

— on soumet à dégradation spécifique des bases, de manière connue en soi, le fragment de découpage marqué et l'on analyse de façon connue en soi les produits de dégradation.